# EUROPEAN PATENT SPECIFICATION

(11) **EP 1 868 450 B1**
(45) Date of publication and mention of the grant of the patent: **22.07.2009**
(21) Application number: 06761896.7
(22) Date of filing: 12.04.2006
(51) Int. Cl.: A23L 1/09, A23L 1/307, A23L 1/29

(54) **SUSTAINED ENERGY RELEASE COMPOSITIONS**
ZUSAMMENSETZUNGEN ZUR VERZÖGERTEN ENERGIEFREISETZUNG
COMPOSITIONS A LIBERATION D'ENERGIE SOUTENUE

(30) Priority: 15.04.2005 EP 05252368
(43) Date of publication of application: 26.12.2007
(73) Proprietor: Cargill, Incorporated, Wayzata, MN 55391 (US)
(72) Inventor: VERCAUTEREN, Ronny, Leontina, Marcel, B-9120 Beveren (BE); DENDOOVEN, Els, Ginette, Alexander, B-1850 Grimbergen (BE)
(74) Representative: Knowles, James Atherton
(86) International application number: PCT/EP2006/003357
(87) International publication number: WO 2006/108626

(56) References cited:
- EP-A- 0 404 964
- DE-A1- 2 264 394
- US-A1- 2004 086 615
- US-A1- 2005 008 678
- US-A1- 2005 095 350
- DATABASE WPI Section Ch, Week 200501 Derwent Publications Ltd., London, GB; Class B03, AN 2005-002844 XP002337315 & JP 2004 331576 A (NOEVIR KK) 25 November 2004 (2004-11-25)

## Description

### Technical Field of the Invention

The present invention relates to the use of certain specified carbohydrate materials in the preparation of sustained glucose and energy release food, feed and drink compositions and to compositions containing such materials.

### Background of the Invention

Carbohydrates are formed of monomer units such as glucose, fructose and galactose. Many carbohydrates are linked to each other by α-1,4-glucosidic bonds that are easily hydrolysed during the early stages of digestion in both humans and animals (i.e. in the mouth, stomach and small intestine). Examples of such carbohydrates include the commercially available hydrolysis products of starch, such as maltose and maltodextrins. It is common knowledge that oral ingestion of such carbohydrates leads to a rapid increase in blood glucose concentration and therefore to an elevated insulin response. This is typically followed by a sharp decrease in blood glucose and, because insulin levels remain high, can result in so called "rebound hypoglycaemia". Symptoms of hypoglycaemia include nausea, weakness, hallucinations, headaches, hypothermia and fainting.

Conversely, for some individuals (such as diabetics), an increase in blood glucose (hyperglycaemia) can be maintained for a prolonged period of time.. This occurs when insulin levels are insufficient to stimulate glucose uptake by tissues (resulting in a normalisation of blood glucose) or when tissue insulin sensitivity is impaired. Such prolonged hyperglycaemia induces undesired effects on metabolism in the body often referred to as metabolic syndrome, a combination of several disease risk factors such as elevated blood pressure, impaired glucose tolerance, elevated fasting blood glucose and impaired blood lipid levels, often together with weight gain. Being exposed to these risk factors is known to lead to a significant increase in morbidity and mortality.

There is therefore a clear need to develop alternative carbohydrate compounds that can be safely ingested whilst retaining the desirable properties (such as sweetness) of more conventional carbohydrates (e.g. glucose, maltose, maltodextrins and sucrose).

A number of high intensity sweeteners have been proposed for helping to reduce the increase in blood glucose, when being substituted for absorbable glucose sources, including, for example, aspartame, saccharin, sucralose or cyclamate. Unfortunately, these sweeteners cannot be used as effective sources of glucose and energy required for maintenance of a normal cellular function of the central nervous system and red blood cells. Moreover, there are conditions in which a consistent supply of glucose is an important characteristic, e.g. in nutritional compositions used by highly active people (such as sports men and women) or by certain categories of patients that have a particularly high energy turnover (burn patients, for example).

From JP 2004331576 the use of glucopyranosyl glycerol for inhibiting rapid blood glucose elevation is known. US 2004/0086615 discloses low calorie confectioner compositions comprising erythritol and a fructo-digosaccaride. EO 0 404964 discloses the production of sugar compounds such as glucopyranosyl erythritol.

The problem to be solved by the present invention is therefore the provision of a carbohydrate material that does not cause rebound hypoglycaemia whilst nevertheless having high sweetness and being a consistent source of glucose and energy.

Attempts have already been made in the art to address this problem. Proposed solutions include carbohydrates such as isomaltulose, certain dextrans and pullulan. All of these carbohydrates, however, have a number of drawbacks. Taking isomaltulose by way of example, although its digestion results in a slow release of glucose into the blood it only has a relatively low sweetness (42% the sweetness of sucrose), it is not very heat or acid stable (stability being a particularly desirable characteristic for compounds used in food compositions) and it has a relatively high Maillard reactivity (meaning that it can lead to undesirable browning).

The present invention therefore aims to provide an alternative to these known, slow-release carbohydrates which does not suffer from the drawbacks associated with the

### prior art.

### Statements of the Invention

In a first aspect of the present invention, there is provided the use of a glucopyranosyl tetritol in the preparation of a sustained energy release food, feed or drink composition.

In a second aspect of the present invention, there is provided a sustained energy release food, feed or drink composition characterised in that it comprises a glucopyranosyl tetritol.

In a third aspect of the present invention, there is provided a process for the preparation of a sustained energy release food, feed or drink composition characterised in that it comprises the step of adding a glucopyranosyl tetritol to said composition.

### Brief Description of the Drawings

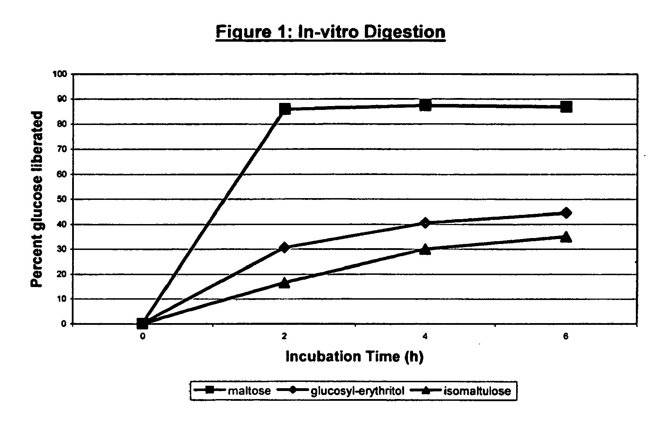

**Figure 1** shows the in-vitro digestibility of O-α-D-Glucopyranosyl erythritol obtained according to Example 3.

### Detailed Description of the Invention

As noted above, the present invention provides, in a first aspect, the use of a glucopyranosyl tetritol in the preparation of a sustained energy release food, feed or drink composition.

### Glucopyranosyl tetritols

Glucopyranosyl tetritols are carbohydrate compounds composed of a glucose unit and C4 sugar alcohol unit. Sugar alcohols (also known as polyols) are derived from carbohydrates whose carbonyl group has been reduced to a primary or secondary hydroxyl group. They have the general formula CₙH₂ₙ+₂Oₙ.

The glucopyranosyl tetritol of the present invention will preferably be selected from the group consisting of glucopyranosyl erythritol, glucopyranosyl D-threitol, glucopyranosyl L-threitol and mixtures of two or more thereof. Most preferably, the glucopyranosyl tetritol will be glucopyranosyl erythritol, preferably in the form O-α-D-glucopyranosyl erythritol.

A number of isomers of each glucopyranosyl tetritol exist, depending on which alcohol group of sugar alcohol the glucose unit is linked to. Thus, for example, O-α-D-glucopyranosyl erythritol can exist as 1-O-α-D-glucopyranosyl erythritol (when the glucose unit is linked to the primary alcohol group of erythritol) or as 2-O-α-D-glucopyranosyl erythritol (when the glucose unit is linked to the secondary alcohol group of erythritol). Preferably, the glucopyranosyl tetritols of the present invention will be in their primary form (i.e. with the glucose unit linked to the primary alcohol group of the sugar alcohol). Accordingly, in a most preferred embodiment, the glucopyranosyl tetritol of the present invention will be 1-O-α-D-glucopyranosyl erythritol.

### Sustained energy release

Glucopyranosyl sugar alcohols, such as glucopyranosyl erythritol, are known in the art. They were thought, however, to be indigestible or only very slightly digestible. They were therefore characterised as being low calorie compounds (see EP0404964, for instance) with only a small proportion of the glucose units ingested being released into the blood-stream. In fact, we have now surprisingly found that glucopyranosyl tetritols, and glucopyranosyl erythritol in particular, undergo a slow but total or near-total hydrolysis in the small intestine resulting, for several hours after ingestion, in a continuous low-level release of glucose into the blood. This effect is referred to herein as "sustained energy release".

Thus, the present invention further provides a sustained energy release food, feed or drink composition characterised in that it comprises a glucopyranosyl tetritol.

### Food, Feed and Drink Compositions

Such compositions could be used, for example, to deliver carbohydrates to diabetic patients without causing clinically significant hyperglycaemia. They could also be added to the diet of overweight or elderly people suffering from reduced glucose, tolerance. In the domain of sports nutrition, glucopyranosyl tetritols could be used to supply athletes with a steady and constant carbohydrate supply during physical exercise. They could also be used in foods or supplements for growing children and in so-called "energy drinks" or "energy bars". This is, of course, a non-exhaustive list and many other potential embodiments of the present invention will be apparent to the skilled person.

### Process for the preparation of sustained energy release compositions

In a further aspect, the present invention provides a process for the preparation of a sustained energy release food, feed or drink composition characterised in that it comprises the step of adding a glucopyranosyl tetritol to said composition.

The glucopyranosyl tetritol will typically be added in an amount of at least 5% by weight. Preferably, it will be added in an amount of at least 15%, even more preferably in an amount of at least 20% by weight based on the total weight of the composition. The exact amount to be added will of course depend on various factors - such as type of application (food, feed or drink), target consumer (sports people, young children, diabetics, etc.), desired level of sweetness and caloric value of the final composition, etc. - and will easily be calculated by the skilled person.

The glucopyranosyl tetritol may be added to the food, feed or drink composition at any stage during its production. Advantageously, glucopyranosyl tetritols have been found to have a good level of sweetness. Typically therefore, the glucopyranosyl tetritol will be added in lieu of or in combination with other carbohydrate materials or sweeteners.

### Advantages of the Invention

It has surprisingly found that, despite not causing any sudden sharp increase in blood glucose concentration, glucopyranosyl tetritols can nonetheless be used as a sustained energy release ingredient. Such sustained supply of energy is known to result in hormonal patterns that favour the feeling of fullness (satiation) and induce less hunger. In addition, glucopyranosyl tetritols have been found to have good sweetness and low cariogenicity when compared to sucrose. Their ingestion leads to only low glycemic and insulinemic responses and therefore presents a significantly reduced risk, if any, of causing either rebound hypoglycaemia or persistent strong hyperglycaemia. A low insulinemic response is also known to promote a high rate of fatty acid mobilisation from adipose (fat) tissue resulting in a high rate of fatty acid oxidation in energy metabolism (Newsholme E.A., Leech A.R. (eds): Integration of carbohydrate and lipid metabolism. In: Biochemistry for the medical sciences. John Wiley & Sons, Chichester 1983, pp 336-35, Newsholme E.A., Start C. (eds): Adipose tissue and the regulation of fat metabolism. In: Regulation in Metabolism. John Wiley & Sons, Chichester 1973a, pp 195-246).

These features make glucopyranosyl tetritols an ideal alternative to other carbohydrates or sweeteners for use in:
- food, feed and drink compositions aimed at supporting physical and mental performance (e.g. in fitness/sports nutrition, infant nutrition, nutrition for the elderly, in so-called "brain foods" directed, for example, at students or people whose jobs require consistently high levels of concentration and/or alertness such as pilots);
- food, feed and drink compositions aimed at individuals requiring slow but continuous carbohydrate energy delivery, for example in conditions that require medical nutrition and/or enteral feeding (e.g. for cachexia patients, burn patients), or for post-operative nutrition;
- food, feed and drink compositions aimed at inducing satiety, reducing overall energy intake and/or increasing fat metabolism (e.g. slimming products, drinks for children, etc.); and
- food, feed and drink compositions aimed at individuals suffering from particular metabolic disorders (such as diabetes, low glucose tolerance and metabolic syndrome (syndrome X) patients).

The present invention shall now be described by way of the following examples.

### Examples

### Example 1: Synthesis of O-α-D-Glucopyranosyl erythritol

A mixture of 58 g water, 40 g erythritol, 100 g maltose and 2 g of transglucosidase (AMANO) was prepared. The pH was adjusted to 4.6 with hydrochloric acid. The mixture was then heated to 50°C for 24h. After filtration and passing through a strong cation exchanger followed by a weak anion exchanger (elution with demi-water), the syrup was vacuum concentrated to 35% dry substance.

The total yield of O-α-D-glucopyranosyl erythritol was 18% as compared to the starting materials (using HPLC analysis).

### Example 2: Purification of O-α-D-glucopyranosyl erythritol

5ml of the mixture obtained in Example 1 was diluted to 25% Brix and loaded on 1.5l Bio-Gel Polyacrylamide P2-fine (BIORAD) resin and eluted with demineralised water at 1.5ml/min at room temperature. 170mg *O*-α-D-glucopyranosyl erythritol was obtained at a purity of 86%.

### Example 3: In-vitro digestibility of O-α-D-glucopyranosyl erythritol

The enriched O-α D-glucopyranosyl erythritol of Example 2 was used as substrate in in-vitro digestibility studies.

1% substrate solutions (w/w) of maltose (from Merck), isomaltulose (from ICN) and O-α-D-glucopyranosyl erythritol were prepared in a 0.05 M phosphate buffer at pH 6 and equilibrated at 37°C for 10 minutes. A suspension of 30% rat intestinal acetone powder (supplied by Sigma) was prepared in 0.05M phosphate buffer (from Merck) at pH 6 and equilibrated at 37°C for 10 minutes.

0.6 ml rat intestinal acetone powder suspension was added to 6 ml of each of the substrate solutions and mixed. The mixtures were incubated at 37°C and a 1 ml sample was taken (0 hours incubation time). Further samples were taken after 2, 4 and 6 hours of incubation. The samples were diluted with 4 ml of demineralised water and boiled for 5 minutes. After the denaturation step, each sample was filtered through a 0.45 µm filter.

The filtrate was send through a Dionex OnGuard-ATM filter. Glucose content was determined by HPLC.

The results for the in-vitro small intestinal digestion tests for maltose, isomaltulose and O-c-D-glucopyranosyl erythritol are given in table 1.

**Table 1 : Percent glucose liberated during in-vitro digestion**

| **incubation time (h)** | **0** | **2** | **4** | **6** |
|---|---|---|---|---|
| **maltose** | 0 | 86 | 87.5 | 87 |
| **O-α-D-glucopyranosyl erythritol** | 0 | 30.5 | 40.5 | 44.5 |
| **isomaltulose** | 0 | 16.5 | 30 | 35 |

It is clear form these results that O-α-D-glucopyranosyl erythritol is digested, in-vitro, at a substantially lower rated than maltose. The digestion rate is in fact close to that of isomaltulose which is known in the art as being a sustained energy release carbohydrate.

## Claims

1. Use of a glucopyranosyl tetritol in the preparation of a sustained energy release food, feed or drink composition, wherein the sustained energy release results from the glucopyranosyl tetritol undergoing hydrolysis in the small intestine resulting in a continuous low-level release of glucose for several hours after ingestion.

2. Use according to claim 1 wherein the glucopyranosyl tetritol is selected from the group consisting of glucopyranosyl erythritol, glucopyranosyl D-threitol, glucopyranosyl L-threitol and mixtures of two or more thereof.

3. Use according to claim 1 or claim 2 wherein the glucopyranosyl tetritol is 1-O-α-D-glucopyranosyl erythritol.

4. Use according to any ane of claims 1-3 wherein the glucopyranosyl tetritol is added in an amount of at least 5% by weight based on the total weight of the sustained energy release food, feed or drink composition.

5. A sustained energy release food, feed or drink compositon **characterised in that** it comprises a glucopyranosyl tetritol, wherein the sustained energy release results from the glucopyranosyl tetritol undergoing hydrolysis in the small intestine resulting in a continuous low-level release of glucose for several hours after ingestion.

6. Process for the preparation of a sustained energy release food, feed or drink composition **characterised in that** it comprises the step of adding a glucopyranosyl tetritol to said composition, wherein the sustained energy release results from the glucopyranosyl tetritol undergoing hydrolysis in the small intestine resulting in a continuous low-level release of glucose for several hours after ingestion.

## Patentansprüche

1. Verwendung von Glucopyranosyltetritol bei der Herstellung einer Nahrungsmittel-, Futtermittel- oder Getränkezusammensetzung mit anhaltender Energiefreisetzung, wobei die anhaltende Energiefreisetzung daraus resultiert, dass das Glucopyranosyltetritol im Dünndarm einer Hydrolyse unterzogen wird, was in einer kontinuierlichen Freisetzung von Glucose in einer niedrigen Konzentration über mehrere Stunden nach der Aufnahme resultiert.

2. Verwendung nach Anspruch 1, wobei das Glucopyranosyltetritol aus der Gruppe bestehend aus Glucopyranosylerythritol, Glucopyranosyl-D-threitol, Glucopyranosyl-L-threitol und Gemischen von zwei oder mehr dieser Verbindungen ausgewählt ist.

3. Verwendung nach Anspruch 1 oder 2, wobei es sich bei dem Glucopyranosyltetritol um 1-0-α-D-Glucopyranosylerythritol handelt.

4. Verwendung nach einem der Ansprüche 1 - 3, wobei das Glucopyranosyltetritol in einer Menge von mindestens 5 Gew.-%, bezogen auf das Gesamtgewicht der Nahrungsmittel-, Futtermittel- oder Getränkezusammensetzung mit anhaltender Energiefreisetzung, zugegeben wird.

5. Nahrungsmittel-, Futtermittel- oder Getränkezusammensetzung mit anhaltender Energiefreisetzung, **dadurch gekennzeichnet, dass** sie ein Glucopyranosyltetritol umfasst, wobei die anhaltende Energiefreisetzung daraus resultiert, dass das Glucopyranosyltetritol im Dünndarm einer Hydrolyse unterzogen wird, was in einer kontinuierlichen Freisetzung von Glucose in einer niedrigen Konzentration über mehrere Stunden nach der Aufnahme resultiert.

6. Verfahren zur Herstellung einer Nahrungsmittel-, Futtermittel- oder Getränkezusammensetzung mit anhaltender Energiefreisetzung, **dadurch gekennzeichnet, dass** es den Schritt des Zugebens eines Glucopyranosyltetritols zu der Zusammensetzung umfasst, wobei die anhaltende Energiefreisetzung daraus resultiert, dass das Glucopyranosyltetritol im Dünndarm einer Hydrolyse unterzogen wird, was in einer kontinuierlichen Freisetzung von Glucose in einer niedrigen Konzentration über mehrere Stunden nach der Aufnahme resultiert.

## Revendications

1. Utilisation de glucopyranosyl tétritol dans la préparation d'une composition alimentaire, alimentaire pour animaux ou buvable à libération d'énergie prolongée, dans laquelle la libération d'énergie prolongée résulte de l'hydrolyse du glucopyranosyl tétritol dans l'intestin grêle qui conduit à une libération continue de faible niveau de glucose pendant plusieurs heures après l'ingestion.

2. Utilisation selon la revendication 1, dans laquelle le glucopyranosyl tétritol est sélectionné parmi le groupe constitué de glucopyranosyl érythritol, de glucopyranosyl D-thréitol, de glucopyranosyl L-thréitol et de mélanges de deux ou plus parmi ceux-ci.

3. Utilisation selon la revendication 1 ou la revendication 2, dans laquelle le glucopyranosyl tétritol est le 1-O-α-D-glucopyranosyl érythritol.

4. Utilisation selon l'une quelconque des revendications 1 à 3, dans laquelle le glucopyranosyl tétritol est ajouté en une quantité d'au moins 5 % en poids par rapport au poids total de la composition alimentaire, alimentaire pour animaux ou buvable à libération d'énergie prolongée.

5. Composition alimentaire, alimentaire pour animaux ou buvable à libération d'énergie prolongée, **caractérisée en ce qu'**elle comprend un glucopyranosyl tétritol, dans laquelle la libération d'énergie prolongée résulte de l'hydrolyse du glucopyranosyl tétritol dans l'intestin grêle qui conduit à une libération continue de faible niveau de glucose pendant plusieurs heures après l'ingestion.

6. Procédé pour la préparation d'une composition alimentaire, alimentaire pour animaux ou buvable à libération d'énergie prolongée, **caractérisé en ce qu'**il comprend l'étape d'ajout d'un glucopyranosyl tétritol à ladite composition, dans lequel la libération d'énergie prolongée résulte de l'hydrolyse du glucopyranosyl tétritol dans l'intestin grêle qui conduit à une libération continue de faible niveau de glucose pendant plusieurs heures après l'ingestion.
